(19) Europäisches Patentamt
European Patent Office
Office européen des brevets

(11) EP 3 064 940 B1

(12) EUROPEAN PATENT SPECIFICATION

(45) Date of publication and mention of the grant of the patent:
**03.07.2019 Bulletin 2019/27**

(51) Int Cl.:
**G01N 33/574** *(2006.01)*

(21) Application number: **14858507.8**

(22) Date of filing: **28.10.2014**

(86) International application number:
**PCT/JP2014/078671**

(87) International publication number:
**WO 2015/064594 (07.05.2015 Gazette 2015/18)**

(54) **SALIVARY BIOMARKER FOR PANCREATIC CANCER**

SPEICHELBIOMARKER FÜR BAUCHSPEICHELDRÜSENKREBS

MARQUEUR BIOLOGIQUE SALIVAIRE POUR CANCER DU PANCRÉAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2013 JP 2013223738**

(43) Date of publication of application:
**07.09.2016 Bulletin 2016/36**

(73) Proprietor: **Salivatech Co., Ltd.**
**Yamagata 997-0052 (JP)**

(72) Inventors:
• **SUGIMOTO, Masahiro**
**Tsuruoka-shi**
**Yamagata 997-0017 (JP)**
• **SOGA, Tomoyoshi**
**Tsuruoka-shi**
**Yamagata 997-0017 (JP)**
• **SUNAMURA, Makoto**
**Tokyo 160-8402 (JP)**

(74) Representative: **Grünecker Patent- und Rechtsanwälte**
**PartG mbB**
**Leopoldstraße 4**
**80802 München (DE)**

(56) References cited:
• **MASAHIRO SUGIMOTO ET AL: "Capillary electrophoresis mass spectrometry-based saliva metabolomics identified oral, breast and pancreatic cancer-specific profiles", METABOLOMICS, KLUWER ACADEMIC PUBLISHERS-PLENUM PUBLISHERS, NL, vol. 6, no. 1, 10 September 2009 (2009-09-10), pages 78-95, XP019796459, ISSN: 1573-3890 -& M Sugimoto: "Figure S3 Sugimoto et al. Choline-Creatinine ratio", METABOLOMICS, 10 September 2010 (2010-09-10), XP055401353, Retrieved from the Internet: URL:https://static-content.springer.com/es m/art:10.1007/s11306-009-0178-y/MediaObjec ts/11306_2009_178_MOESM3_ESM.ppt [retrieved on 2017-08-25] -& M Sugimoto: "Table S2 Sugimoto et al. Salivary metabolites and the ratio of the relative area of oral, breast and pancreatic cancers, and periodontal diseases to controls.", METABOLOMICS, 10 September 2010 (2010-09-10), XP055401354, Retrieved from the Internet: URL:https://static**
• **ZHANG L ET AL: "Salivary Transcriptomic Biomarkers for Detection of Resectable Pancreatic Cancer", GASTROENTEROLOGY, ELSEVIER, AMSTERDAM, NL, vol. 138, no. 3, 1 March 2010 (2010-03-01) , pages 949-957.e7, XP026995494, ISSN: 0016-5085 [retrieved on 2009-11-18]**

EP 3 064 940 B1

- FARRELL J J ET AL: "964 Multiple Salivary Biomarkers for Pancreatic Cancer Detection", GASTROENTEROLOGY, ELSEVIER, AMSTERDAM, NL, vol. 136, no. 5, 1 May 2009 (2009-05-01), pages A-147, XP026111209, ISSN: 0016-5085, DOI: 10.1016/S0016-5085(09)60662-4 [retrieved on 2009-05-01]
- MASAHIRO SUGIMOTO ET AL.: 'Capillary electrophoresis mass spectrometry-based saliva metabolomics identified oral, breast and pancreatic cancer-specific profiles' METABOLOMICS vol. 6, 2010, pages 78 - 95, XP019796459
- TAKAO ITOI ET AL.: 'Metabolome Kaiseki ni yoru Suigan Kenshin eno Aratana Kanosei' JAPANESE ASSOCIATION FOR CANCER DETECTION AND DIAGNOSIS vol. 20, no. 1, 14 July 2012, page 50, XP008183837
- TAKAO ITOI ET AL.: 'Daeki Metabolome Kaiseki o Mochiita Suigan Shindanho no Kaihatsu' JOURNAL OF THE JAPAN PANCREAS SOCIETY vol. 27, no. 3, 30 May 2012, page 397, XP008183662

## Description

The present invention relates to a diagnosis method for identifying a person suffering from pancreatic cancer.

A treatment for pancreatic cancer, which is intractable cancer, has not yet been sufficient, and the median survival year has not yet reached a year for a non-surgical excision example of pancreatic cancer, such as chemotherapy and radiotherapy. That is, in order to improve these treatment results of intractable cancer, cancer diagnosis at an early stage in which surgical excision is still possible is important. Thus, by using a biological sample(body fluid, etc.) that can be easily collected minimally or non-invasively, testing is frequently performed, and a method needs to be developed, which can detect cancer at an early stage or at a stage in which excision treatment is still possible at the latest.

In Patent Literature 1, one of the present inventors proposes a serum marker for determining a kidney disease. In Patent Literatures 2 and 3, he proposes a liver disease marker.

Additionally, for early pancreatic cancer detection, many procedures are also used, which detect a tumor diagnosis marker from blood (Patent Literatures 4 and 5). As a protein marker in blood related to cancer of a digestive system, carbohydrate antigen 19-9 (CA19-9) is used in a clinical field and is useful so as to detect pancreatic cancers and biliary tract cancers as well as to evaluate effects of chemotherapy. However, early cancer diagnosis is difficult, and the accuracy of screening cancer is insufficient (Non-Patent Literature 1). In addition, among negative people of the Lewis blood group system, there is a problem that false negatives are shown in which the level of CA19-9 is not elevated even if it is cancer. Additionally, detection of DUPAN-2 (pancreatic cancer associated antigen) and CEA (CarcinoEmbryonic Antigen) are also available. However, the former shows positive for biliary tract and liver cancers, and the latter also shows positive even for the cancers of the digestive system, such as esophageal cancer, and gastric cancer. Therefore, these markers are not specific to pancreatic cancer. Further, these markers have not been widely used due to costs.

Polyamines, such as spermine (spermine), and acetylated polyamines, such as N8-acetylspermidine (N8-Acetylspermidine), N1-acetylspermidine (N1-Acetylspermidine), and N1-acetylspermine (N1-Acetylspermine) are known as markers for various cancers in blood and urine (Non-Patent Literature 2). In a metabolic pathway, arginine is metabolized to ornithine, and then metabolized through putrescine to polyamines. Biosynthesis of these is activated in a place in which a large amount of oxygen exists at a suface of a cancer tissue. In a hypoxic state at a center of cancer, uptake from outside of a cell increases. It is considered that as an entire cancer tissue, the concentration becomes high and flows into blood. For example, an increase in the concentration of spermidine in blood is known in breast cancers, prostate cancers and testis tumors (Non-Patent Literature 1). Furthermore, it is known through animal testing that the concentrations of spermine and spermidine in blood decrease in acute pancreatitis (Non-Patent Literature 3).

Citation List

Patent Literature

Patent Literature 1: Japanese Patent Application Laid-Open No. 2011-58863
Patent Literature 2: Japanese Patent Application Laid-Open No. 2011-232164
Patent Literature 3: WO2011/158590A1
Patent Literature 4: Japanese Patent Application Laid-Open No. 2011-247869
Patent Literature 5: Japanese Translation of PCT International Application No. 2009-508493, i.e. JP2009508493 (A).
Patent Literature 6: Japanese Patent Application Laid-Open No. 2013-521763

Non-Patent Literature

Non-Patent Literature 1: Hamada S, Shimosegawa T., Biomarkers of pancreatic cancer, Pancreatology. 2011; 11: 14-9
Non-Patent Literature 2: Soda K (2011), The mechanisms by which polyamines accelerate tumor spread. Journal of Experimental & Clinical Cancer Research. 30(1): 95
Non-Patent Literature 3: Jin HT, Lamsa T, Merentie M, Hyvonen MT, Sand J, Raty S, Herzig KH, Alhonen L, Nordback I (2008). Polyamine levels in the pancreas and the blood change according to the severity of pancreatitis. Pancreatology. 8(1), 15-24
Non-Patent Literature 4: Zhang L, Farrell JJ, Zhou H, Elashoff D, Akin D, Park NH, Chia D, Wong DT. (2010), Salivary transcriptomic biomarkers for detection of resectable pancreatic cancer. Gastroenterology. 138(3): 949-57

Non-Patent Literature 5: Sugimoto M, Wong DT, Hirayama A, Soga T, Tomita M, (2010), Capillary electrophoresis mass spectrometry-based saliva metabolomics identified oral, breast and pancreatic cancer-specific profiles, Metabolomics, 6, 78-95

Non-Patent Literature 6: Soga, T., Baran, R., Suematsu M., Ueno, Y., Ikeda, S., Sakurakawa T., Kakazu, Y., Ishikawa, T., Robert, M., Nishioka, T., Tomita, M. (2006), Differential methabolomics reveals ophthalmic acid as an oxidative stress biomarker indicating hepatic glutathione sonsumption., Journal of Biological Chemistry, 281 (24): 16768-16776

Non-Patent Literature 7: Sugimoto et al. Capillary electrophoresis mass spectrometry-based saliva metabolomics identified oral, breast and pancreatic cancer-specific profiles, Metabolomics, 2010, 6, 78-95

Non-Patent Literature 8: Tsutsui et al, High-throughput LC-MS/MS based simultaneous determination of polyamines including N-acetylated forms in human saliva and the diagnostic approach to breast cancer patients, Anal Chem, 2013, 85, 11835-42

Non-Patent Literature 9: Wang Q et al. Investigation and identification of potential biomarkers in human saliva for the early diagnosis of oral squamous cell carcinoma., Clin Chim Acta. 2014; 427: 79-85

Conventional detection of cancer using protein in blood is insufficient for screening for early pancreatic cancer detection. Additionally, although a blood test is a minimally invasive test, for a health examination or at a hospital, a blood sample is needed, which is collected by a healthcare professional, who uses a syringe. Thus, it is actually difficult to perform a test very frequently. In contrast, using saliva is non-invasive and does not cause any pain at all. There is an advantage that regardless of the location, even an individual subject can collect saliva and that it is possible to perform a test very frequently. For example, a salivary biomarker for detecting lung cancer isproposed in Patent Literature 6. In particular, it is said that early pancreatic cancer detection is difficult. It is important to detect a possibility of cancer promptly, using a test, which is performed much more frequently than a blood test.

As for a possibility of diagnosing pancreatic cancer by using saliva, Non-Patent Literature 4 discloses that pancreatic cancer can be diagnosed by using mRNA in saliva. Furthermore, there is also a report that pancreatic cancer can be diagnosed by using a metabolite in blood.

However, for mRNA quantitation, a step is needed in which an RNase inhibitor is added to saliva immediately after collecting saliva such that mRNA is not destroyed. Additionally, a complex step and time is required for measurement. Because of this, quantitative PCR is used for quantitation. However, in general, approximately several markers are only quantitated. In Non-Patent Literature 4 as well, 35 substances are only quantitated. Thus, trends of many molecules cannot be comprehensively and quantitatively understood by holding exhausitivity and quantitative property, and the saliva concentration cannot be corrected. Therefore, highly accurate prediction is impossible. Furthermore,, the step is complex, so there is a high possibility that artificial noise may be mixed with a quantitative value. In the past, there was no proposal that reports a marker search example with high reliability that also considers effects of a concentration fluctuation, which is generated in saliva, by minimizing a possibility of mixing noise with a quantitative value by means of a simpler processing, and comprehensively quantitating molecules. Additionally, a diagnosis method using such a salivary biomarker with high liability was not proposed, which was able to distinguish a healthy person from a person with cancer, particularly pancreatic cancer, intraductal papillary mucinous neoplasm (IPMN), breast cancer, and oral cancer.

The present invention was made to solve the above conventional problems. An object of the present invention is to be able to detect cancer, such as pancreatic cancer, at an early stage, using saliva.

The present invention provides a diagnosis method for identifying a person suffering from pancreatic cancer, comprising the steps of:

detecting the concentration of creatinine, NI-acetylspermidine, $\alpha$-aminoadipic acid, N-acetylneuraminic acid, and 1,3-diaminopropane in a collected saliva sample of a person,

comparing the detected concentration of creatinine, N1-acetylspermidine, $\alpha$-aminoadipic acid, N-acetylneuraminic acid, and 1,3-diaminopropane with standard values,

determining whether there are significant differences in the concentration of creatinine, N1-acetylspermidine, $\alpha$-aminoadipic acid, N-acetylneuraminic acid, and 1,3-diaminopropane from standard values,

attributing a saliva sample showing such significant differences to a person suffering from pancreatic cancer.

The present inventors identified multiple biomarkers that simultaneously quantitate low molecules (metabolites) in saliva of pancreatic cancer patients and discriminate pancreatic cancer patients from healthy controls by using CE-MS combining Capillary Electrophoresis (CE) and -Mass Spectrometry (MS) that can comprehensively measure the low molecules of biological samples. Additionally, they combined these and developed a discriminating method with higher accuracy than a single marker and also evaluated specificity by using saliva of cancer other than saliva of pancreatic cancer. A method of collecting saliva is made uniform, and the collection is performed by eliminating effects of diets and changes during the day, but even so, concentration variations cannot be completely eliminated. Thus, a marker was also searched, which suggests the total of metabolite concentrations, and algorithm was developed, which is combined with a marker that discriminates pancreatic cancer patients from healthy controls.

The detection of pancreatic cancer is possible with a high accuracy by using a combination of creatinine, N1-acetylspermidine, α-aminoadipic acid, N-acetylneuraminic acid and 1,3-diaminopropane according to the claim. Prediction can also be made by using another combination or changing the methodology of the combination thereof.

The present invention provides a method according to the claim, said method involving assaying a salivary biomarker for pancreatic cancer including the steps of: collecting a saliva sample; and detecting the salivary biomarker for pancreatic cancer in the collected saliva sample.

The present specification further discloses a device for assaying a salivary biomarker for cancer including means for collecting a saliva sample, and means for detecting the aforementioned salivary biomarker for cancer in the collected saliva sample.

Here, when the salivary biomarker for cancer is selected, correlation values of the measured metabolites are calculated, and a concentration of each substance can be normalized, using a concentration of a substance that is correlated to the largest number of substances.

A combination of the salivary biomarkers for cancer can be determined using a mathematical model.

Advantageous Effects of Invention

According to the present invention, pancreatic cancer can be detected early, using saliva that can be collected non-invasively and simply. In particular, a combination of polyamine with another novel substance makes a highly accurate prediction possible.

FIG. 1 is a flowchart illustrating the procedure for determining the biomarkers used in the Examples of the present invention.

FIG. 2 is a diagram illustrating a correlation network between metabolites in saliva used in the Examples.

FIG. 3 is a flowchart illustrating a procedure of developing a mathematical model used in the Examples.

FIG. 4 is a diagram illustrating a model of a decision tree that distinguishes a subject with pancreatic cancer from a healthy subject.

FIG. 5 is a diagram illustrating a receiver operating characteristic (ROC) curve of a mathematical model that distinguishes a subject with pancreatic cancer from a healthy subject using a metabolite concentration normalized with a concentration marker used in the Examples.

FIG. 6 is a diagram in which a risk of pancreatic cancer (PC) for a healthy subject (C), and subjects with pancreatic cancer (PC), chronic pancreatitis (CP), and IPMN is plotted in a model of classifying the healthy subject and the subject with pancreatic cancer in the Examples.

FIG. 7 is a diagram illustrating a stepwise forward selection method used for variable selection in an MLR model that distinguishes a subject with pancreatic cancer from a healthy subject when the absolute concentration of the concentration marker as used in the Examples.

FIG. 8 is a diagram illustrating a forward selection method used for variable selection in the MLR model that distinguishes a subject with pancreatic cancer from a healthy subject when the absolute concentration of the concentration marker as used in the Examples.

FIG. 9 is a diagram illustrating an example of a total concentration of amino acids in saliva used in the Examples.

Hereinafter, an embodiment suitably implementing the present invention (hereinafter referred to as the embodiment) will be described in detail.

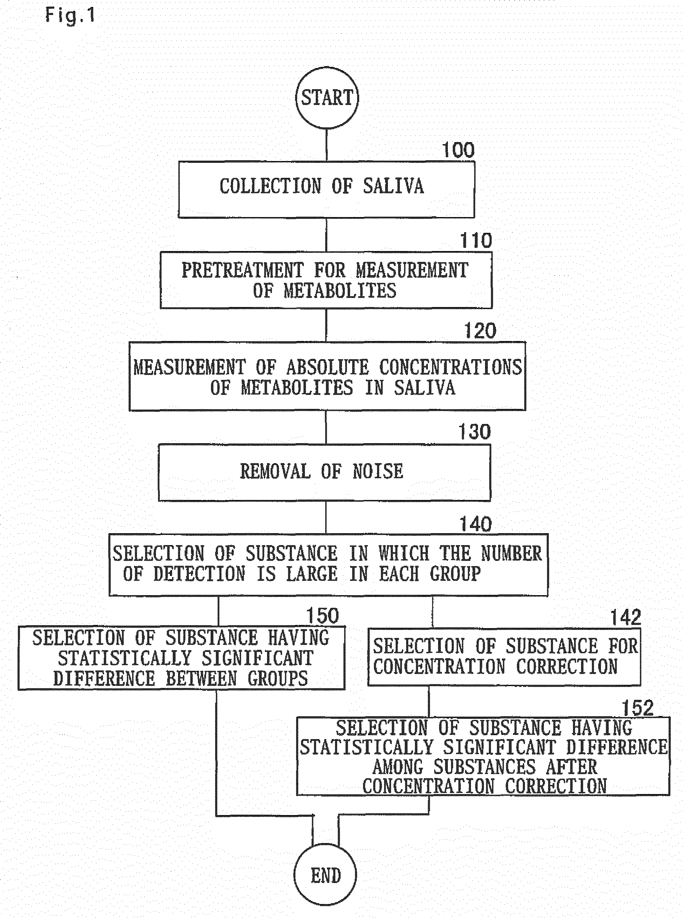

A procedure of determining a biomarker for a pancreatic disease will be described with reference to FIG. 1.

1. Saliva donor

A total of 199 saliva samples was collected from (i) patients with pancreatic cancer at various stages, (ii) healthy controls, and (iii) patients with intraductal papillary mucinous neoplasm (IPMN) and chronic pancreatitis for evaluation of specificity. Table 1 lists the number of cases, the number of males and females, and age in each group. Cases in which patients never experienced chemotherapy before treatment began were subject to this procedure. The number of deficiencies of Table 1 shows the number of cases in which values are unknown.

Table 1

| DISEASE | STAGE | THE NUMBER OF CASES | SEX | | | AGE | | | |
|---|---|---|---|---|---|---|---|---|---|
| | | | MALE | FEMALE | THE NUMBER OF DEFECTS | MINIMUM | MEDIAN | MAXIMUM | THE NUMBER OF DEFECTS |
| HEALTHY | - | 63 | 50 | 13 | | 19 | 43 | 73 | |
| CHRONIC PANCREATITIS | - | 14 | 12 | 2 | | 32 | 49 | 79 | |
| IPMN | - | 7 | 6 | 1 | | 63 | 64 | 69 | 2 |
| PANCREATIC CANCER | I | 3 | 1 | 2 | | 61 | 68 | 76 | |
| | II | 3 | 1 | 1 | 1 | 67 | 67.5 | 68 | 1 |
| | III | 18 | 10 | 8 | | 51 | 69.5 | 95 | |
| | Iva | 37 | 14 | 16 | 7 | 45 | 69 | 85 | 7 |
| | Ivb | 54 | 30 | 22 | 2 | 43 | 72 | 86 | 3 |
| | SUBTOTAL OF PANCREATIC CANCER | 115 | 56 | 49 | 10 | 43 | 70 | 95 | 11 |

2. Method for collecting saliva (Step 100 in FIG. 1)

∘ With respect to collection date
Collection is performed on a day other than a surgery day as much as possible.
∘ With respect to diet
After 21:00 of the day before the collection, do not drink anything but water.
On the day of collection, do not eat breakfast.
∘ Notes before collection of saliva on the day
Collect saliva from AM 8:30 to 11:00 before breakfast.
Brush teeth without use of toothpaste 1 hour or more before the collection of saliva. Do not strenuously exercise 1 hour before the collection of saliva.
Do not clean the inside of oral cavity (with a toothpick, etc.).
Do not smoke.
Do not drink anything but water.
∘ Method for collecting saliva

The mouth is rinsed with water before the collection of saliva, and non-irritant mixed saliva is collected.

Only saliva that runs spontaneously but is not volitionally generated is collected (sialemesis method). Alternatively, a straw is placed in the mouth when saliva is retained to some extent in the mouth (the time is about 3 minutes), and the saliva runs into a tube (passive drool method). When the face is turned down and saliva in the mouth is pushed into the straw that is vertically set, the saliva is likely to run spontaneously. However, when the saliva adheres to a middle of the straw and does not fall down, the saliva is sent out by the breath (in this case, saliva is easily collected by retaining saliva in the mouth to some extent and then pushing the saliva into the tube at one time as compared with opening of the mouth to the tube).

200 μL or more of saliva (as much as possible) is collected.

During the collection of saliva, the tube is placed on ice and kept at a low temperature as much as possible, and the collection is finished within 15 minutes (even when 200 μL of saliva is not collected, the collection is finished in 15 minutes).

Within 5 minutes, the saliva is cryopreserved on ice at -80°C or with dry ice for storage. The tube and the straw of collecting saliva is a tube and a straw made of a polypropylene material.

A method for collecting saliva is not limited to the aforementioned method, and another method may be used.

3. Pretreatment method for measurement of metabolites in saliva (Step 110 in FIG. 1)

400 μL of saliva sample is taken, placed in an ultrafiltration filter (molecular weight cutoff: 5,000 Da), and centrifuged at 4°C and 9,100 g for 3.5 hours. 45 μL of the filtrate and 5 μL of an aqueous solution in which the concentration of each of methionine sulfone (Methionine sulfone), 2-morpholinoethanesulfonic acid monohydrate (2-Morpholinoethanesulfonic acid, monohydrate), CSA (D-Camphor-10-sulfonic acid), sodium salt, 3-aminopyrrolidine (3-Aminopyrrolidine), and trimesic acid (Trimesate) is 2 mM are mixed to prepare 50 μL of a sample. Measurement was performed by the following method.

4. Measurement of absolute concentrations of metabolites in saliva by capillary electrophoresis-time-of-flight mass spectrometry (CE-TOFMS) (Step 120 in FIG. 1)

Ionic metabolites were identified and quantitatively determined from saliva by metabolome analysis using CE-MS.

A measurement method was performed in accordance with the method described in Non-Patent Literature 6. Hereinafter, the parameters will be described.

1) Cationic metabolite measurement mode

• HPCE

Capillary: fused silica, 50 μm in inner diameter × 100 cm in length

Buffer: 1 M formic acid (formate)
Voltage: positive, 30 kV
Temperature: 20°C
Injection: injection under a pressure of 50 mbar for 5 seconds (about 3 nL)
Washing before measurement: with 30 mM ammonium formate (Ammonium Formate) at a pH of 9.0 for 5 minutes, Milli-Q water for 5 minutes, and buffer for 5 minutes

• TOFMS

Polarity: positive
Capillary voltage: 4,000 V
Fragmentor voltage: 75 V
Skimmer voltage: 50 V
OCT RFV: 125 V
Drying gas: nitrogen ($N_2$), 10 L/min
Drying gas temperature: 300°C
Nebulizer gas pressure: 7 psig
Sheath liquid: 50% MeOH / 0.1 $\mu$M Hexakis (2,2-difluoroethoxy) phosphazene-containing water
Flow rate: 10 mL/min
Reference m/z: 2MeOH $^{13}$C isotope [M+H]+m/z 66.063061, Hexakis(2,2-difluoroethoxy)phosphazene [M+H]+m/z 622.028963

2) Anionic metabolite measurement mode

• HPCE

Capillary: COSMO (+), 50 $\mu$m in inner diameter $\times$ 10.6 cm in length
Buffer: 50 mM ammonium acetate, pH: 8.5
Voltage: negative, 30 kV
Temperature: 20°C
Injection: injection under a pressure of 50 mbar for 30 seconds (about 30 nL)
Washing before measurement: with 50 mM ammonium acetate at a pH of 3.4 for 2 minutes, and 50 mM ammonium acetate at a pH of 8.5 for 5 minutes

• TOFMS

Polarity: negative
Capillary voltage: 3,500 V
Fragmentor voltage: 100 V
Skimmer voltage: 50 V
OCT RFV: 200 V
Drying gas: nitrogen ($N_2$), 10 L/min
Drying gas temperature: 300°C
Nebulizer gas pressure: 7 psig
Sheath liquid: 5 mM ammonium acetate and 50% MeOH / 0.1 $\mu$M Hexakis (2,2-difluoroethoxy) phosphazene-containing water
Flow rate: 10 mL/min
Reference m/z: 2[$CH_3COOH$] $^{13}$C isotope [M-H]-m/z 120.038339, Hexakis(2,2-difluoroethoxy) phosphazene + $CH_3COOH$ [M-H]- 680.035541
ESI needle: platinum

The anionic metabolite measurement may be performed before the cationic metabolite measurement.

5. Removal of noise (Step 130 in FIG. 1)

Signals of a substance in which a value largely varies depending on a measurement day and a substance not derived from a metabolite are removed.

From measurement data, all peaks in which a signal noise ratio was 1.5 or more were first detected. A commercially available standard substance was measured before measurement of the saliva samples. A peak in which a value of mass to charge ratio (m/z) obtained by a mass spectrometer and a corresponding migration time were assigned to a substance name. Thus, identification was performed. In quantitative determination, the peak area of each peak was divided by the area of the peak of the internal standard substance, a fluctuation of measurement sensitivity of the mass spectrometer was corrected, and the specific peak area ratio was calculated. The absolute concentration was calculated from a ratio of the specific peak area in the saliva samples to the specific peak area of the standard substance.

6. Selection of substance detected highly frequently in each group (Step 140 in FIG. 1)

Only a substance in which the peak can be detected in 30% or more cases (for example, three out of ten) of each group was selected.

7. Selection of a substance having a statistically significant difference between groups (Step 150 in FIG. 1)

After a typical test (in this case, Mann-Whitney test) was performed, a P value was corrected using a false discovery rate (FDR) and a Q value was calculated. A substance having a significant difference of $Q < 0.05$ was selected.
The substance selected by this procedure is a substance described in Claim 3.

8. Selection of substance for presuming concentrations of all metabolites in saliva and performing concentration correction (Step 142 in FIG. 1)

In all the samples measured (including healthy, breast cancer, oral cancer, IPMN, and pancreatic cancer), correlation values between the metabolites were exhaustively calculated using the determined quantitative values of the metabolites. Combinations of substances satisfying a Pearson correlation coefficient (R) of $R \geq 0.8$ were listed. Of a metabolite group in which the most substances correlated with each other, a substance that correlated with the most substances was selected.

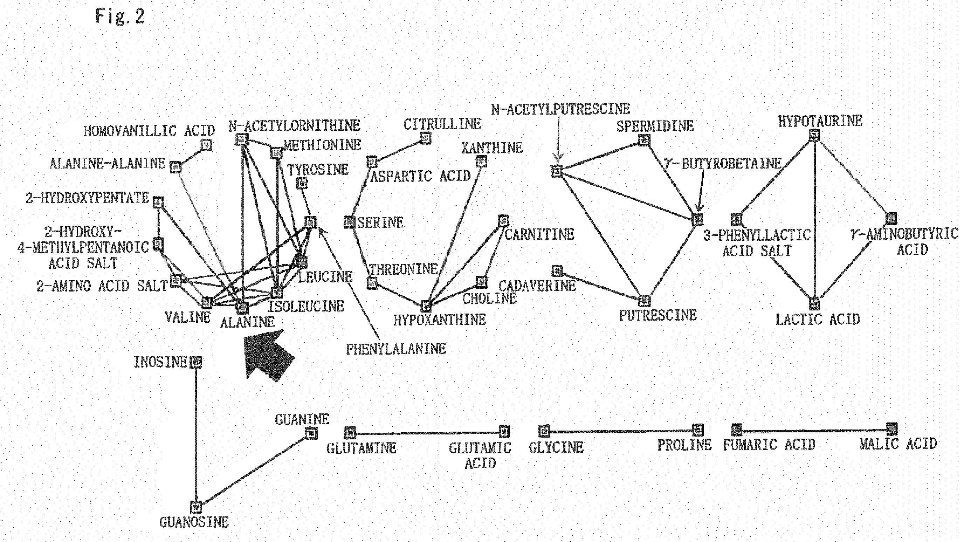

FIG. 2 shows one example of a correlation network diagram of the metabolites in saliva.

9. Selection of a substance having a statistically significant difference among the substances after concentration correction (Step 152 in FIG. 1)

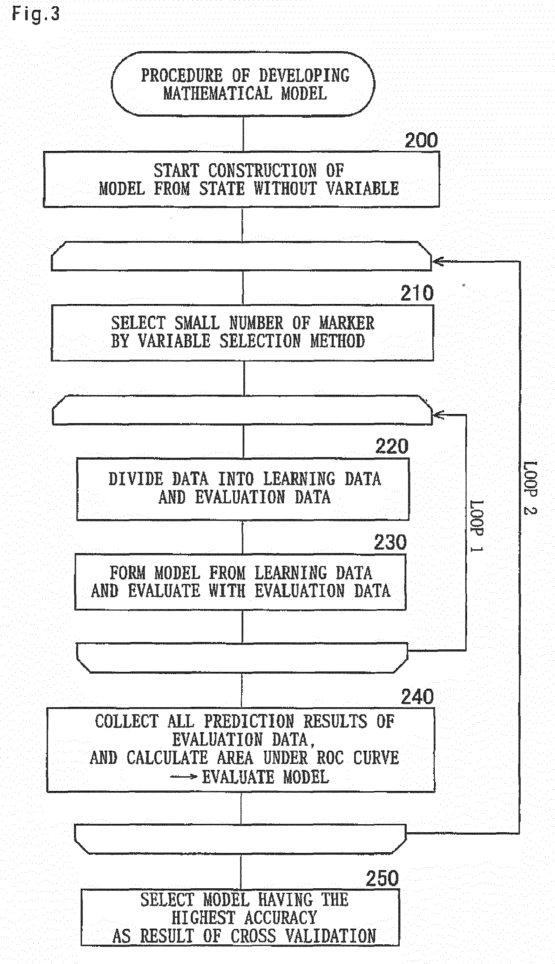

After the typical test (in this case, Mann-Whitney test) was performed using a value in which the concentration of each substance was corrected with the concentration of the substance selected at Step 142, a P value was corrected using the false discovery rate (FDR), and a Q value was calculated. A substance having a significant difference of $Q < 0.05$ was selected.
A procedure of developing a mathematical model of distinguishing the subjects with pancreatic cancer from the healthy subjects will be then described with reference to FIG. 3.
Using the marker selected at Step 150 or 152 in FIG. 1, a multiple logistic regression model (MLR model) that is a mathematical model was developed from a state in which a variable did not exist at Step 200. In the analysis of multiple logistic regression (MLR), a regression equation of P that is

$$\ln(P/1 - P) = b_0 + b_1 x_1 + b_2 x_2 + b_3 x_3 + \cdots + b_k x_k \ (1)$$

is determined using k description variables $x_1$, $x_2$. $x_3$, ..., and $x_k$ for a ratio P as a target variable.
Specifically, a combination of the smallest independent variables that did not correlate with each other was selected at Step 210, for example, using a stepwise forward selection method of stepwise variable selection. A P value at which the variable was added was 0.05, a P value at which the variable was eliminated was 0.05, and a variable $x_i$ was selected.
At Step 220, the data were divided into learning data and evaluation data, and at Step 230, a model was formed from the learning data and evaluated using the evaluation data. In cross validation of Loop 1 in FIG. 3, Steps 220 and 230 were repeated.

At Step 240, receiver operating characteristic (ROC) analysis was performed using the selected model. An area under the ROC curve (AUC) and a 95% confidential interval (CI) were calculated, and the model was evaluated. In accordance with the ROC curve, a curve of $Y = X + \alpha$ ($\alpha$ is a constant) was drawn. When the value of $\alpha$ was decreased from 1 to 0, the value of $\alpha$ that first touched the ROC curve was determined. Thus, an optimal cut-off value was determined.

Next, the process proceeded to Step 250, and a model having the best accuracy as the result of cross validation was selected.

Herein, a stepwise method is used. The stepwise method includes three kinds of a forward selection method, a stepwise forward selection method, and a backward selection method. The threshold value may be adjusted to a threshold value of $P < 0.05$, and variable may be added. Therefore, the model having the best accuracy can be selected by forming a model many times at a larger loop 2 in FIG. 3.

Specifically, for evaluation of the MLR model, values of risk of pancreatic cancer (PC) with respect to saliva of breast cancer, oral cancer (CP), and IPMN were calculated. A group of the healthy subjects (C), and the subjects with CP and IPMN was formed. An AUC value that could identify pancreatic cancer from this group was calculated. The data were randomly divided into 10, a model was formed using 90% of the data, and the model was evaluated by the rest values of 10%. This operation was repeated 10 times. All the cases were selected once for evaluation, and cross validation (CV) of collecting the evaluation data and calculating the AUC value was performed.

10. Results of model of distinguishing pancreatic cancer from searched substance

FIG. 2 shows substances that exhibited high correlation values with the metabolites quantitatively determined at Step 120 in FIG. 1 at Step 142. In FIG. 2, a line is drawn between substances having $R \geq 0.8$. Eight clusters (groups of metabolites) are confirmed, but a cluster on the far left upper side in the drawing contains the most substances. In the cluster, alanine (Ala) forms the most networks with other substances. Therefore, alanine is determined as a metabolite for normalizing the concentration of the whole saliva. The metabolite used for normalization is not limited to the substance forming the most networks with other substances. For example, the total concentration of the metabolites, the sum of signals obtained during measurement of saliva by CE-MS (total ion electropherogram), or the area of a peak that is at a central order when all detected signals are sorted according to size may be used for normalization. The variable selection and the mathematical model are not limited to the stepwise method and the MLR model, respectively.

For example, for the variable selection, a correlation-based feature subset method (see M. A. Hall (1998). Correlation-based Feature Subset Selection for Machine Learning. Hamilton, New Zealand.), a relief method (see Marko Robnik-Sikonja, Igor Kononenko (1997). An adaptation of Relief for attribute estimation in regression. In: Fourteenth International Conference on Machine Learning, 296-304.), an SVM valiable selection method (see I. Guyon, J. Weston, S. Barnhill, V. Vapnik (2002). Gene selection for cancer classification using support vector machines. Machine Learning. 46(1-3): 389-422.), or the like may be applied.

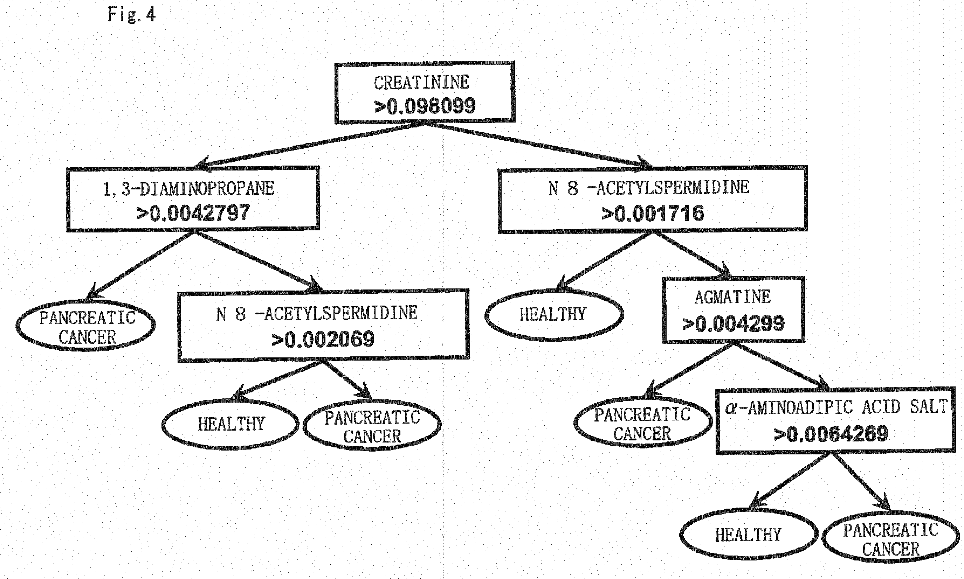

For the mathematical model, a mechanical learning method of dividing two groups may be applied. For example, Bayesian estimate (see Berger, James O (1985). Statistical Decision Theory and Bayesian Analysis. Springer Series in Statistics (Second ed.). Springer-Verlag. ISBN 0-387-96098-8.), neural network (ANN) (see D. E. Rumelhart, G. E. Hinton, and R. J. Williams, (1986): Learning representaions by back-propagating errors, Nature, 323-9, 533-536.), support vector machine (SVM) (see J. Platt (1998) Fast Training of Support Vector Machines using Sequential Minimal Optimization. In B. Schoelkopf and C. Burges and A. Smola, editors, Advances in Kernel Methods-Support Vector Learning), Alternative decision tree (ADTree) (see Yoav Freund and Llew Mason (1999) The Alternating Decision Tree Algorithm. Proceedings of the 16th International Conference on Machine Learning, 124-133, and Freund, Y., Mason, L. (1999) The alternating decision tree learning algorithm. In: Proceeding of the Sixteenth International Conference on Machine Learning, Bled, Slovenia, 124-133), decision tree (see Ross Quinlan (1993). C4.5: Programs for Machine Learning. Morgan Kaufmann Publishers, San Mateo, CA), PART model (see Eibe Frank, Ian H. Witten (1998) Generating Accurate Rule Sets Without Global Optimization. In: Fifteenth International Conference on Machine Learning, 144-151), Random forest, PLS discriminant analysis (see Partial least squares-discriminant analysis; PLS-DA)(Lindgren, F; Geladi, P; Wold, S (1993). The kernel algorithm for PLS. J. Chemometrics 7: 45-59. doi: 10.1002/cem.1180070104.), Orthogonal PLS discriminant analysis (OPLS-DA) (see Trygg, J., & Wold, S. (2002). Orthogonal projections to latent structures (O-PLS). Journal of Chemometrics, 16(3), 119-128, and Breiman, Leo (2001). Random Forests. Machine Learning 45 (1): 5-32. doi: 10.1023/A: 1010933404324.), or the like may be applied. Bootstrap method and Bagging method (see Breiman, Leo (1996) Bagging predictors. Machine, Learning24 (2): 123-140) in which prediction is performed using average and majority of predictive values of a plurality of mathematical models that are obtained by forming a plurality of mechanical learning methods of dividing two groups may be used. Further, separation may be performed using a principal component in principal component analysis (Principal Component Analysis; PCA) (see Hotelling, H. (1933). Analysis of a complex of statistical variables into principal components. Journal of Educational Psychology, 24, 417-441) that is unsupervised learning. FIG. 4 is a model of decision tree that distinguishes the subjects with pancreatic cancer from the healthy subjects. The concentrations of metabolites that were normalized with a concentration marker (in this case, Ala) were used. The area under the ROC curve was 0.856 and the area under the ROC curve during 10-fold cross validation was 0.653.

Substances having a high ability of distinguishing the subjects with pancreatic cancer from the healthy subjects at Step 152 of the above section 9 are shown in Table 2.

Table 2

| NAME OF SUBSTANCE | CONCENTRATION NORMALIZED WITH ALA (NO UNIT) | | | | DETECTION RATIO (%) | | MANN-WHITNEY TEST | | ROC CURVE | | |
|---|---|---|---|---|---|---|---|---|---|---|---|
| | HEALTHY SUBJECT | | PANCREATIC CANCER | | HEALTHY SUBJECT | PANCREATIC CANCER | P VALUE | Q VALUE | AREA | 95% CI | P VALUE |
| | AVERAGE | STANDARD DEVIATION | AVERAGE | STANDARD DEVIATION | | | | | | | |
| N8-ACETYLSPERMIDINE | 0.0015 | 0.0015 | 0.0049 | 0.0071 | 70 | 90 | 1.55773E-06 | 0.000190043 | 0.7175 | 0.6414 to 0.7936 | 0.0001 |
| CREATININE | 0.1793 | 0.1349 | 0.1026 | 0.0946 | 100 | 99 | 1.74565E-05 | 0.001064849 | 0.695 | 0.6140 to 0.7759 | 0.0001 |
| SPERMINE | 0.0129 | 0.0222 | 0.0319 | 0.0477 | 63 | 83 | 3.99507E-05 | 0.001624661 | 0.6852 | 0.6036 to 0.7669 | 0.0001 |
| ASPARTIC ACID | 0.5559 | 0.2454 | 0.4290 | 0.2803 | 98 | 100 | 9.5103E-05 | 0.002320512 | 0.6772 | 0.5965 to 0.7578 | 0.0001 |
| N1-ACETYLSPERMIDINE | 0.0237 | 0.0147 | 0.0407 | 0.0351 | 97 | 99 | 0.000141561 | 0.002878407 | 0.6727 | 0.5929 to 0.7526 | 0.0001424 |
| N1-ACETYLSPERMINE | 0.0019 | 0.0045 | 0.0046 | 0.0060 | 30 | 60 | 7.37102E-05 | 0.002248182 | 0.6679 | 0.5868 to 0.7490 | 0.0002175 |
| CYTIDINE | 0.0232 | 0.0320 | 0.0107 | 0.0198 | 79 | 56 | 0.000175296 | 0.00305516 | 0.6663 | 0.5828 to 0.7499 | 0.0002494 |
| α-AMINOADIPIC ACID | 0.0381 | 0.0234 | 0.0689 | 0.0811 | 97 | 99 | 0.000784335 | 0.010632096 | 0.6525 | 0.5712 to 0.7337 | 0.0007859 |
| CYTOSINE | 0.0042 | 0.0094 | 0.0105 | 0.0180 | 35 | 61 | 0.000495928 | 0.007562907 | 0.6489 | 0.5662 to 0.7316 | 0.001037 |
| BETAINE | 0.2236 | 0.3431 | 0.1548 | 0.4206 | 98 | 98 | 0.001209678 | 0.013416426 | 0.6469 | 0.5614 to 0.7325 | 0.001211 |
| UREA | 39.1920 | 43.7834 | 23.5791 | 42.6852 | 98 | 98 | 0.001345148 | 0.013675675 | 0.6455 | 0.5595 to 0.7316 | 0.001347 |
| HOMOVANILLIC ACID | 0.0653 | 0.1133 | 0.0999 | 0.1260 | 46 | 75 | 0.00108606 | 0.013249934 | 0.645 | 0.5573 to 0.7327 | 0.001404 |
| N-ACETYLNEURAMINIC ACID | 1.6138 | 1.8792 | 0.9916 | 1.3150 | 92 | 95 | 0.00158931 | 0.014559781 | 0.6433 | 0.5560 to 0.7307 | 0.001592 |
| CYSTINE | 0.0040 | 0.0062 | 0.0068 | 0.0071 | 43 | 70 | 0.001670795 | 0.014559781 | 0.6381 | 0.5526 to 0.7236 | 0.002351 |
| UROCANIC ACID | 0.0965 | 0.0780 | 0.0896 | 0.0826 | 98 | 97 | 0.003836014 | 0.029249607 | 0.6313 | 0.5458 to 0.7187 | 0.003834 |
| FUMARIC ACID | 0.0104 | 0.0209 | 0.0206 | 0.0294 | 35 | 59 | 0.00304094 | 0.024732975 | 0.6262 | 0.5418 to 0.7105 | 0.005451 |
| 1, 3-DIAMINOPROPANE | 0.0516 | 0.0554 | 0.0304 | 0.0397 | 78 | 73 | 0.00511095 | 0.034103773 | 0.6261 | 0.5368 to 0.7154 | 0.005477 |
| HYPOTAURINE | 0.0322 | 0.0508 | 0.0498 | 0.0739 | 51 | 78 | 0.004991032 | 0.034103773 | 0.6255 | 0.5352 to 0.7157 | 0.005712 |
| NICOTINIC ACID | 0.2849 | 1.1623 | 0.1163 | 0.5318 | 75 | 65 | 0.005311243 | 0.034103773 | 0.6246 | 0.5365 to 0.7127 | 0.006067 |
| AGMATINE | 0.0050 | 0.0050 | 0.0033 | 0.0039 | 81 | 69 | 0.005639892 | 0.034403344 | 0.6244 | 0.5377 to 0.7112 | 0.006123 |
| VALINE | 0.3794 | 0.2213 | 0.4894 | 0.3424 | 100 | 99 | 0.006972284 | 0.040119018 | 0.6225 | 0.5380 to 0.7070 | 0.006964 |
| 2-HYDROXY-4-METHYLPENTANOIC ACID | 0.0645 | 0.0686 | 0.0949 | 0.0948 | 75 | 94 | 0.007234577 | 0.040119018 | 0.6218 | 0.5333 to 0.7103 | 0.007288 |
| ALANYL-ALANINE | 0.0301 | 0.0175 | 0.0247 | 0.0139 | 89 | 96 | 0.010929763 | 0.055559628 | 0.6155 | 0.5261 to 0.7049 | 0.01092 |
| CITRIC ACID | 0.3689 | 0.5593 | 0.2466 | 0.5154 | 95 | 85 | 0.014695008 | 0.067709323 | 0.6107 | 0.5265 to 0.6949 | 0.01474 |
| GLUCOSAMINE | 0.0124 | 0.0117 | 0.0078 | 0.0104 | 65 | 55 | 0.01143552 | 0.055805337 | 0.6106 | 0.5209 to 0.7004 | 0.0148 |
| CARNOSINE | 0.0029 | 0.0046 | 0.0016 | 0.0040 | 54 | 38 | 0.008103017 | 0.042981222 | 0.609 | 0.5195 to 0.6985 | 0.01629 |
| GLYCINE-GLYCINE | 0.0154 | 0.0158 | 0.0229 | 0.0202 | 56 | 80 | 0.015539845 | 0.067709323 | 0.6086 | 0.5203 to 0.6968 | 0.01677 |
| 2-AMINOBUTYRIC ACID | 0.0756 | 0.1971 | 0.0723 | 0.0719 | 95 | 100 | 0.017659224 | 0.074290531 | 0.6077 | 0.5190 to 0.6965 | 0.01762 |
| ARGININE | 0.5163 | 0.3841 | 0.4082 | 0.4195 | 100 | 99 | 0.019323999 | 0.078584264 | 0.6062 | 0.5197 to 0.6927 | 0.01928 |
| N-ACETYLGLUTAMIC ACID | 0.0035 | 0.0057 | 0.0053 | 0.0060 | 40 | 63 | 0.015062166 | 0.067709323 | 0.6052 | 0.5172 to 0.6933 | 0.02041 |
| GLYCEROPHOSPHORIC ACID | 0.3663 | 0.2880 | 0.3039 | 0.3335 | 100 | 99 | 0.020954571 | 0.082466378 | 0.6048 | 0.5208 to 0.6889 | 0.02091 |
| PHOSPHOENOLPYRUVIC ACID | 0.0130 | 0.0194 | 0.0222 | 0.0398 | 44 | 67 | 0.026459721 | 0.100877686 | 0.5972 | 0.5093 to 0.6852 | 0.03216 |
| ISOLEUCINE | 0.1286 | 0.0693 | 0.1629 | 0.1046 | 100 | 100 | 0.037465083 | 0.138507278 | 0.5945 | 0.5089 to 0.6800 | 0.03738 |
| ADENOSINE | 0.0126 | 0.0131 | 0.0093 | 0.0141 | 81 | 78 | 0.039739367 | 0.142594198 | 0.593 | 0.5040 to 0.6820 | 0.04054 |
| GUANINE | 0.0520 | 0.0403 | 0.0449 | 0.0543 | 94 | 91 | 0.04241365 | 0.147344023 | 0.5921 | 0.5065 to 0.6778 | 0.04236 |
| DIHYDROXYACETONEPHOSPHORIC ACID | 0.2462 | 0.1806 | 0.2041 | 0.1814 | 94 | 97 | 0.047321948 | 0.154153689 | 0.5901 | 0.5043 to 0.6758 | 0.04722 |
| CADAVERINE | 0.5943 | 0.7614 | 0.9336 | 1.2717 | 100 | 99 | 0.048015084 | 0.154153689 | 0.5898 | 0.5039 to 0.6757 | 0.0479 |

In Table 2, a detection ratio shows a ratio of cases in which a peak can be detected relative to all the cases in each group of the healthy subjects and the subjects with pancreatic cancer. A 95% confidential interval (CI) represents a value of 95% confidential interval.

A Mann-Whitney test that is a non-parametric two-group test for the healthy subject group and the pancreatic cancer group was performed between the healthy subjects and the subjects with pancreatic cancer, the p value of each of the metabolites was calculated, and the p value was corrected with the false discovery rate (FDR). A test of q value was performed.

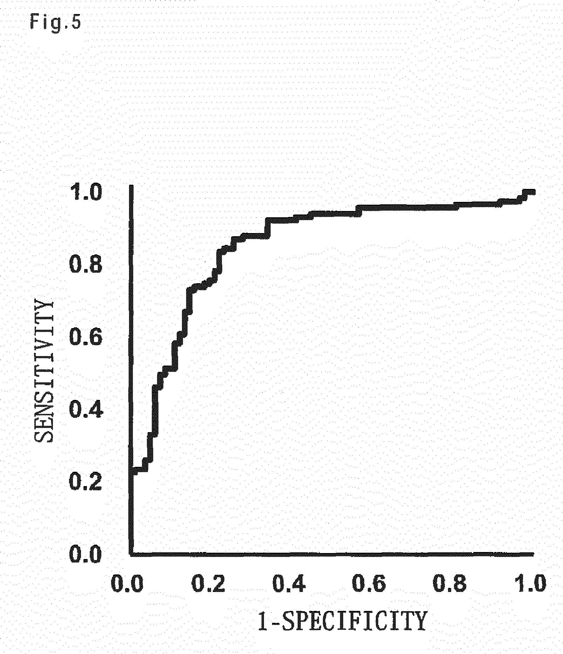

For evaluation of sensitivity and specificity of the substances that distinguish two groups of the subjects with pancreatic cancer (PC) and the healthy subjects (C), receiver operating characteristic (ROC) analysis was performed. The results are shown in FIG. 5. The concentrations of metabolites that were normalized with the concentration marker were used. The substances contained in the MLR model and a parameter and an odds ratio thereof are shown in Table 3.

Table 3

| ITEM | PARAMETER | P VALUE | 95% CI | | ODDS RATIO | 95% CI | |
|---|---|---|---|---|---|---|---|
| (INTERCEPT) | −0.0375549 | 0.9179 | −0.7551751 | 0.67987596 | − | − | − |
| CREATININE | 8.81954122 | <.0001 | 5.11051632 | 13.1970776 | 6765.16 | 165.7559 | 538788.1 |
| N1−ACETYLSPERMIDINE | −36.266806 | 0.0017 | −60.649929 | −14.849848 | 1.78E−16 | 4.57E−27 | 3.56E−07 |
| α−AMINOADIPIC ACID | −25.286236 | 0.0039 | −43.368968 | −9.1982815 | 1.04E−11 | 1.46E−19 | 0.000101 |
| N−ACETYLNEURAMINIC ACID | 0.30754159 | 0.0219 | 0.05238651 | 0.58469864 | 1.360077 | 1.053783 | 1.79445 |
| 1,3−DIAMINOPROPANE | 11.309134 | 0.004 | 4.02635127 | 19.6651022 | 81563.25 | 56.056 | 3.47E+08 |

The area under the ROC curve in FIG. 5 was 0.8763 (95% CI: 0.8209 to 0.9317, p < 0.0001). The sensitivity of optimal cut-off value was 0.8348, and (1-specificity) was 0.2169.

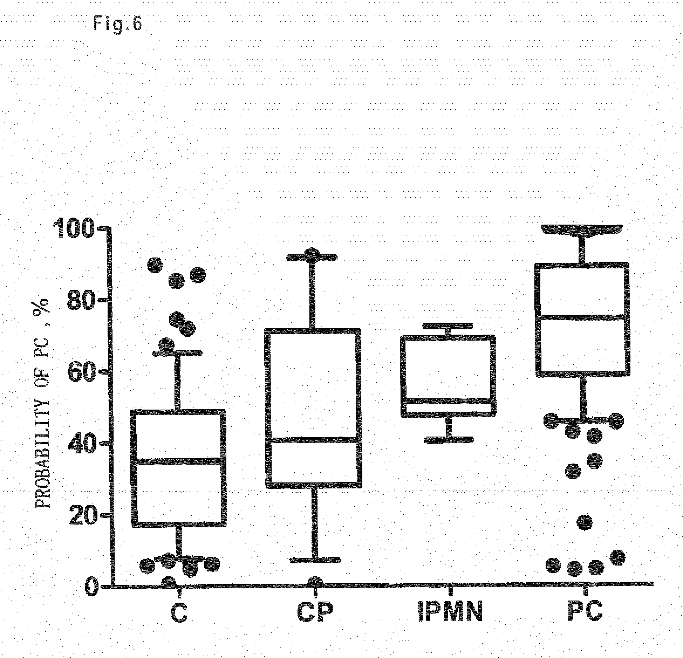

Using the MLR model that can distinguish the healthy subjects and the subjects with pancreatic cancer, values calculated as risk of pancreatic cancer (PC) of the healthy subjects (C) and the subjects with pancreatic cancer (PC) as well as the patients with breast cancer, oral cancer (CP), and IPMN are shown in FIG. 6.

FIG. 6 is a diagram in which the risk of pancreatic cancer (PC) of C, PC, breast cancer, oral cancer (CP), and IPMN is plotted by the model of classifying the healthy subjects (C) and the subjects with pancreatic cancer (PC). A boxplot represents values of 10%, 25%, 50%, 75%, and 90% from the top, and values under 10% and values beyond 90% are expressed as plots.

Table 4 shows an AUC value in which the specificity and general-purpose properties of the MLR model were evaluated.

Table 4

| | DISTINGUISH PC FROM C | DISTINGUISH PC FROM C+CP+IPMN |
|---|---|---|
| WHEN ALL THE DATA ARE USED | 0.88 | 0.85 |
| IN CASE OF CV | 0.86 | 0.83 |

Herein, CV represents a case of cross validation.

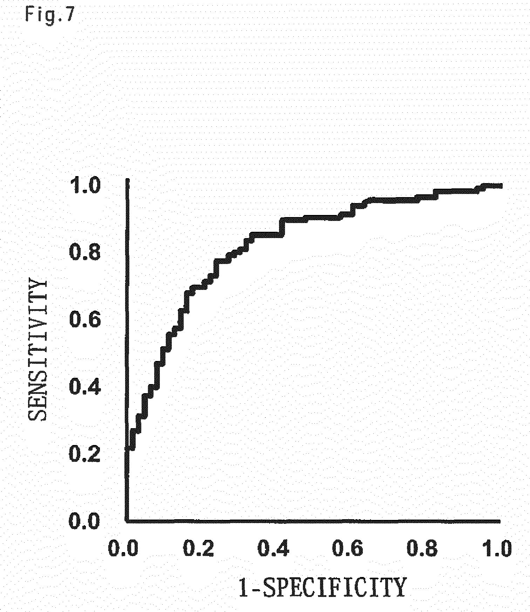

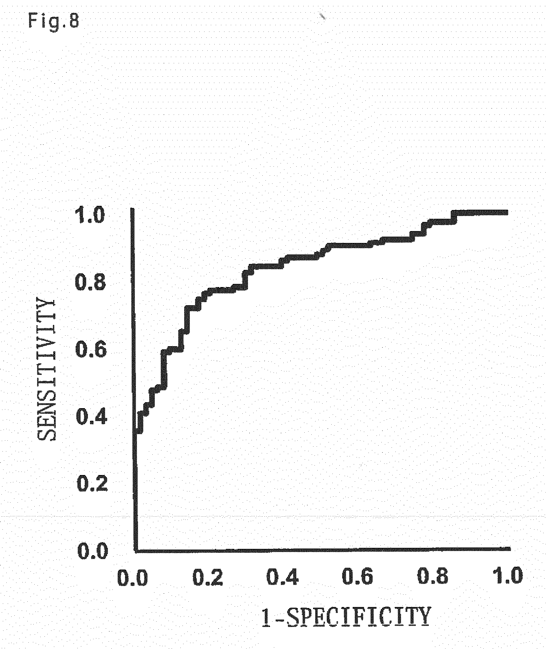

In FIG. 7, an ROC curve at which the MLR model was formed using the absolute concentration without concentration correction is shown. Table 5 shows selected markers and coefficients. The area under the ROC curve was 0.8264 (95% CI: 0.7619 to 0.8874, p < 0.0001). The accuracy was slightly decreased as compared with a case with concentration correction, but highly accurate prediction was possible. For formation of this model, a P value at which the variable was added was 0.05, and a P value at which the variable was eliminated was 0.05 using a stepwise forward selection method of stepwise variable selection. FIG. 8 shows an ROC curve at which the P value at which the variable was added was 0.05 using a forward selection method as a method of variable selection. Table 6 shows selected markers and coefficients. The area under the ROC curve was 0.8373 (95% CI: 0.7792 to 0.8954, p < 0.0001). Regardless of use of the markers and coefficients that were different from the model of FIG. 7, prediction accuracy of the same level could be achieved.

Table 5

| ITEM | PARAMETER | P VALUE | 95% CI | | ODDS RATIO | 95% CI | |
|---|---|---|---|---|---|---|---|
| (INTERCEPT) | 0.40019 | 0.162 | -0.15107 | 0.977355 | - | - | - |
| $\alpha$-AMINOADIPIC ACID | -0.64356 | <.0001 | -0.95539 | -0.39782 | 0.525421 | 0.384661 | 0.671782 |
| PHOSPHORYLCHOLINE | -0.04641 | 0.0427 | -0.09647 | -0.00235 | 0.954646 | 0.90804 | 0.997649 |
| N-ACETYLNEURAMINIC ACID | 0.014466 | <.0001 | 0.007874 | 0.02224 | 1.014571 | 1.007905 | 1.022489 |

Table 6

| ITEM | PARAMETER | P VALUE | 95% CI | | ODDS RATIO | 95% CI | |
|---|---|---|---|---|---|---|---|
| (INTERCEPT) | 0.277503 | 0.3624 | -0.31567 | 0.884388 | - | - | - |
| 3PG | 0.166107 | 0.0028 | 0.063545 | 0.282987 | 1.1807 | 1.065608 | 1.327088 |
| N1-ACETYLSPERMINE | -2.26566 | 0.0127 | -4.28489 | -0.65321 | 0.103762 | 0.013775 | 0.520371 |
| $\alpha$-AMINOADIPIC ACID | -0.9899 | <.0001 | -1.47201 | -0.58351 | 0.371613 | 0.229465 | 0.557935 |
| N-ACETYLNEURAMINIC ACID | 0.012425 | 0.0007 | 0.005686 | 0.020249 | 1.012503 | 1.005702 | 1.020456 |
| 4-($\beta$-ACETYLAMINOETHYL) IMIDAZOLE | 4.469352 | 0.0041 | 1.397495 | 7.786111 | 87.30013 | 4.045055 | 2406.938 |

11. Consideration of searched substance

In the present invention, the concentrations of ionic metabolites contained in saliva were simultaneously measured, and markers having a high ability of distinguishing the subjects with pancreatic cancer from the healthy subjects were selected. Further, a model having higher accuracy (sensitivity and specificity) as compared with a single substance could be developed by combining the markers.

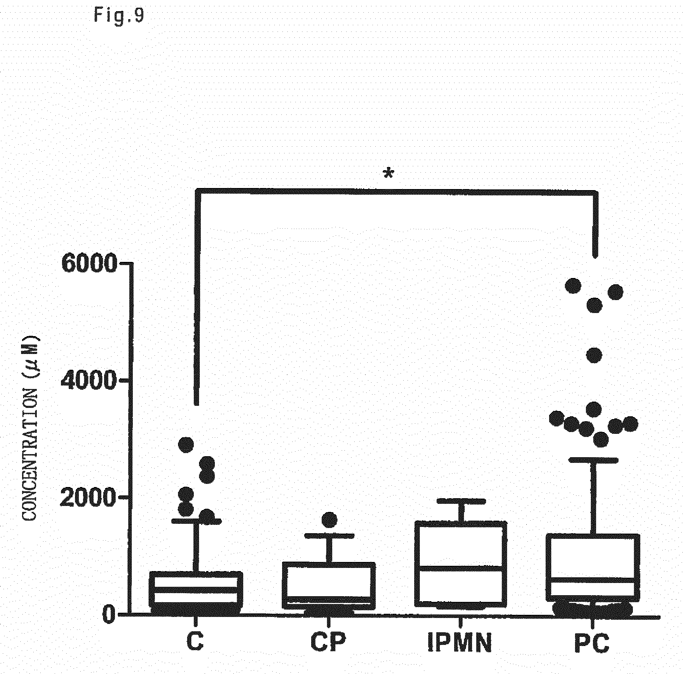

A problem involved in using saliva is that there is a greater variation of concentrations present in saliva as compared with blood. In this method, saliva was collected under unified conditions depending on the collection time and dietary restriction before the collection. Some of the samples had trends in which the concentrations of all the substances were clearly high or low. FIG. 9 shows a difference in the total concentration of amino acids in saliva of each disease. A boxplot has the same meanings as that of FIG. 6. A Kruskal-Wallis test that is a non-parametric multiplex test was performed, and the P value was 0.0138. After that, a Dunn's post test was performed. Only between C and PC, the P value was less than 0.05.

The concentration in the subjects with pancreatic cancer (PC) is significantly higher than that in the healthy subjects (C), and as an indication exhibiting a risk of pancreatic cancer, a high total concentration in saliva itself may be used. However, some of the samples of C have a concentration higher than PC, and in contrast, some of the samples of PC have a concentration lower than C. Therefore, when the samples are simply considered for a risk, the accuracy is low (from results of ROC analysis between C and PC in data of FIG. 8, AUC = 0.6282, and p = 0.004756).

Because of this, if these samples (the concentration is high in C and the concentration is low in PC) are excluded and only samples whose concentrations are within a certain range are subject to the test, the entire concentration can also be considered, but the number of cases that is subject to the test needs to be reduced. Therefore, the fluctuation of the entire concentration was offset by performing normalization using a substance that has a high correlation with the entire metabolite concentration of the saliva and can be detected in all the samples by the method shown in FIG. 2. The normalization may be omitted.

Among the substances as marker candidates in Table 2, polyamines such as spermine, and acetylated polyamines such as N8-acetylspermidine, N1-acetylspermidine, and Nl-acetylspermine are each a substance that reflects on a state of the pancreatic tissues according to various changes in cancer. However, for example, in a case of spermine in urine, the concentration correction with creatinine is only considered. Therefore, spermine cannot achieve the accuracy that a tumor marker measured in a blood test can achieve. Because polyamines in blood are taken up by erythrocytes (see Fu NN, Zhang HS, MaM, Wang H. (2007) Quantification of polyamines in human erythrocytes using a new near-infrared cyanine 1-(epsilon-succinimidyl-hexanoate)-1'-methyl-3,3,3',3'-tetramethyl-indocarbocyanine-5,5'-dis ulfonate potassium with CE-LIF detection. Electrophoresis. 28(5): 822-9), the amount of polyamines in a free state is extremely small, and the concentration thereof in urine is extremely low. Even when polyamines in blood and urine are measured in breast cancer, the highest concentration of spermidine is about 140 nM (nanomol), and the concentration of N-acetylspermidine is about 64 nM (nanomol). Thus, concentrations that are much lower than the concentrations in saliva are reported (see Byun JA, Lee SH, Jung BH, Choi MH, Moon MH, Chung BC. (2008) Analysis of polyamines as carbamoyl derivatives in urine and serum by liquid chromatography-tandem mass spectrometry. Biomed Chromatogr. 22(1): 73-80). The quantitative determination of polyamines in erythrocytes requires a complicated step. Therefore, a diagnosis method found in the present invention has characteristics in which a highly accurate prediction can be achieved due to the contribution of the following three points, including (i) use of saliva capable of detecting the marker substances at high concentration, (ii) a decrease in dispersion generated at each measurement due to a simple treatment process for measurement, and (iii) use of the mathematical model in combination with the markers. A difference in mRNA in saliva between the patients with pancreatic cancer and the healthy subjects is already known (Non-Patent Literature 4). However, mRNA is completely different because a molecular group to which the present invention is directed is a metabolite. The variation of metabolites by themselves in saliva depending on pancreatic cancer is already known (Non-Patent Literature 5). However, substances that are not disclosed in known documents are used as a marker in the present invention, and a mathematical model for eliminating the effect of a specific concentration variation in saliva and identifying pancreatic cancer with high sensitivity and specificity can be developed.

With respect to four groups of healthy subjects, chronic pancreatitis, IPMN, and pancreatic cancer, a distribution of risk of pancreatic cancer that is predicted by the MLR model shows that the model exhibits high specificity for pancreatic cancer (FIG. 6). The results of cross validation (Table 4) and the results of a test for distinguishing the pancreatic cancer group from the groups other than the pancreatic cancer group also show that this model has high sensitivity and specificity that cannot be achieved by the conventional method.

In Examples, capillary electrophoresis-mass spectroscopy (CE-MS) is used to measure the concentrations of metabolites in saliva. However, high speed liquid chromatography (LC), gas chromatography (GC), chip LC, or chip CE, or GC-MS, LC-MS, and CE-MS methods in which they are combined with a mass spectrometer (MS), a measurement method for each MS alone, an NMR method, a measurement method for a metabolite substance that is derivatized into a fluorescent substance or a UV absorptive material, or an enzyme method in which an antibody is produced and measured by an ELISA method, may be used. Regardless of the measurement method, measurement may be performed by any analysis.

Saliva for LC-MS is treated as follows.

1) In 270 $\mu$L of MeOH and NH$_4$OH adjusted to 2 $\mu$M MES, saliva stored at -80°C is dissolved, and 30 $\mu$L thereof is added and stirred.

2) The mixture is centrifuged at 4°C and 15,000 rpm for 10 minutes, and the entire upper layer is transferred to another tube.

3) The whole amount of the liquid is subjected to centrifugal concentration, and added to the liquid are 18 $\mu$L of 90% MeOH and 12 $\mu$L of BorateBuffer, resulting in redissolution.

4) 5 $\mu$L of the liquid is used for LC-MS analysis, and 20 $\mu$L of the liquid is used for ELISA analysis.

5) In the LC-MS analysis, 10 $\mu$L of MilliQ water containing 4 $\mu$M Methionine-sulfone is added to 5 $\mu$L of the aforementioned solution to obtain a dilution as a sample.

Measurement conditions of LC-MS are as follows.

LC system: Agilent Technologies 1290 infinity
Mobile phase: Solvent A; Water containing 1% Formic acid: Solvent B; Acetonitrile containing 0.1% formic acid
Flow rate: 0.5 mL/min
Gradient [min. (%B)]: 0(98)-1(98)-3(55)-5(5)
Stop time: 7 min
Post time: 3 min
Column: CAPCELL CORE PC (Shiseido: 2.1 mm $\times$ 50 mm, 2.7 mm)
Column temp.: 50°C
Injection volume: 1 $\mu$L
MS: Agilent Technologies G6230A
Gas temp: 350°C
Gas flow: 13 L/min
Neblizer Gas: 55 psig
Fragmentor: 150
Skimmer: 90
OCT1 RF Vpp: 200
VCap: 3500
Reference: 121.050873, 922.009798
Mode: Positive

According to the present invention, when the concentration of saliva is corrected (normalized), using data analysis of a correlation network reduces the influence of the concentration. Even in saliva in which concentrations vary greatly, a subject with pancreatic cancer can be distinguished from a healthy subject.

A range in which a test can be performed using the marker of the present invention is determined by the value of concentration-correcting marker that reflects the saliva concentration, and saliva whose overall concentration is outside shoulde be treated as outliers. In saliva within the range, a patient with each cancer can be distinguished from a healthy subject by a mathematical model that combines the markers of absolute concentrations or corrected relative concentrations.

Industrial Applicability

Even by using saliva in which the concentration largely varies, pancreatic cancer can be early detected in a healthy subject.

**Claims**

**1.** A diagnosis method for identifying a person suffering from pancreatic cancer, comprising the steps of:

detecting the concentration of creatinine, N1-acetylspermidine, $\alpha$-aminoadipic acid, N-acetylneuraminic acid, and 1,3-diaminopropane in a collected saliva sample of a person,
comparing the detected concentration of creatinine, N1-acetylspermidine, $\alpha$-aminoadipic acid, N-acetyl-

neuraminic acid, and 1,3-diaminopropane with standard values,
determining whether there are significant differences in the concentration of creatinine, N1-acetylspermidine, $\alpha$-aminoadipic acid, N-acetylneuraminic acid, and 1,3-diaminopropane from standard values,
attributing a saliva sample showing such significant differences to a person suffering from pancreatic cancer.

**Patentansprüche**

1. Diagnoseverfahren zum Identifizieren einer Person, die an Bauchspeicheldrüsenkrebs leidet, umfassend die Schritte:

Ermitteln der Konzentration von Kreatinin, N1-Acetylspermidin, $\alpha$-Aminoadipinsäure, N-Acetylneuraminsäure und 1,3-Diaminopropan in einer gesammelten Speichelprobe einer Person,
Vergleichen der ermittelten Konzentration von Kreatinin, N1-Acetylspermidin, $\alpha$-Aminoadipinsäure, N-Acetyl-neuraminsäure und 1,3-Diaminopropan mit Standardwerten,
Bestimmen, ob signifikante Unterschiede in der Konzentration von Kreatinin, N1-Acetylspermidin, $\alpha$-Aminoa-dipinsäure, N-Acetylneuraminsäure und 1,3-Diaminopropan von den Standardwerten bestehen,
Zuordnen einer Speichelprobe, die solche signifikanten Unterschiede aufweist, zu einer Person, die an Bauchspeicheldrüsenkrebs leidet.

**Revendications**

1. Procédé de diagnostic permettant d'identifier une personne souffrant d'un cancer du pancréas, comprenant les étapes consistant à:

détecter la concentration de créatinine, de N1-acétylspermidine, d'acide $\alpha$-aminoadipique, d'acide N-acétylneuraminique et de 1,3-diaminopropane dans un échantillon de salive prélevé chez une personne,
comparer la concentration détectée de créatinine, de N1-acétylspermidine, d'acide $\alpha$-aminoadipique, d'acide N- acétylneuraminique et de 1,3-diaminopropane à des valeurs standard,
déterminer s'il existe des différences significatives dans les concentrations de créatinine, de N1-acétylspermi-dine, d'acide $\alpha$-aminoadipique, d'acide N-acétylneuraminique et de 1,3-diaminopropane par rapport aux valeurs standard,
attribuer un échantillon de salive présentant de telles différences significatives à une personne atteinte de cancer du pancréas.

Fig.1

```
                        ┌─────────┐
                        │  START  │
                        └─────────┘
                             │
                             │                    100
              ┌──────────────────────────────────┐
              │       COLLECTION OF SALIVA        │
              └──────────────────────────────────┘
                             │
                             │                    110
              ┌──────────────────────────────────┐
              │   PRETREATMENT FOR MEASUREMENT    │
              │         OF METABOLITES            │
              └──────────────────────────────────┘
                             │
                             │                    120
            ┌────────────────────────────────────────┐
            │ MEASUREMENT OF ABSOLUTE CONCENTRATIONS  │
            │        OF METABOLITES IN SALIVA         │
            └────────────────────────────────────────┘
                             │
                             │                    130
              ┌──────────────────────────────────┐
              │          REMOVAL OF NOISE         │
              └──────────────────────────────────┘
                             │
                             │                    140
          ┌──────────────────────────────────────────┐
          │ SELECTION OF SUBSTANCE IN WHICH THE NUMBER│
          │  OF DETECTION IS LARGE IN EACH GROUP      │
          └──────────────────────────────────────────┘
                  │                         │
          150     │                  142    │
    ┌──────────────────────┐    ┌──────────────────────────┐
    │ SELECTION OF SUBSTANCE│    │ SELECTION OF SUBSTANCE FOR│
    │    HAVING             │    │ CONCENTRATION CORRECTION  │
    │ STATISTICALLY SIGNIFI-│    └──────────────────────────┘
    │ CANT DIFFERENCE       │                 │
    │ BETWEEN GROUPS        │          152    │
    └──────────────────────┘    ┌──────────────────────────────┐
                  │             │ SELECTION OF SUBSTANCE HAVING │
                  │             │ STATISTICALLY SIGNIFICANT     │
                  │             │ DIFFERENCE AMONG SUBSTANCES   │
                  │             │ AFTER CONCENTRATION CORRECTION│
                  │             └──────────────────────────────┘
                  │                         │
                  └────────────┬────────────┘
                          ┌─────────┐
                          │   END   │
                          └─────────┘
```

Fig. 2

Fig.3

PROCEDURE OF DEVELOPING
MATHEMATICAL MODEL

200
START CONSTRUCTION OF
MODEL FROM STATE WITHOUT VARIABLE

210
SELECT SMALL NUMBER OF MARKER
BY VARIABLE SELECTION METHOD

220
DIVIDE DATA INTO LEARNING DATA
AND EVALUATION DATA

230
FORM MODEL FROM LEARNING DATA
AND EVALUATE WITH EVALUATION DATA

LOOP 1

LOOP 2

240
COLLECT ALL PREDICTION RESULTS OF
EVALUATION DATA,
AND CALCULATE AREA UNDER ROC CURVE
⟶ EVALUATE MODEL

250
SELECT MODEL HAVING THE
HIGHEST ACCURACY
AS RESULT OF CROSS VALIDATION

Fig. 4

CREATININE
>0.098099

N 8 –ACETYLSPERMIDINE
>0.001716

1,3–DIAMINOPROPANE
>0.0042797

AGMATINE
>0.004299

α–AMINOADIPIC ACID SALT
>0.0064269

N 8 –ACETYLSPERMIDINE
>0.002069

HEALTHY

PANCREATIC CANCER

PANCREATIC CANCER

HEALTHY

PANCREATIC CANCER

PANCREATIC CANCER

HEALTHY

PANCREATIC CANCER

Fig.5

Fig.6

Fig.7

Fig.8

Fig.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011058863 A
- JP 2011232164 A
- WO 2011158590 A1
- JP 2011247869 A
- JP 2009508493 W
- JP 2009508493 A
- JP 2013521763 A

### Non-patent literature cited in the description

- **HAMADA S ; SHIMOSEGAWA T.** *Biomarkers of pancreatic cancer, Pancreatology,* 2011, vol. 11, 14-9
- **SODA K.** The mechanisms by which polyamines accelerate tumor spread. *Journal of Experimental & Clinical Cancer Research,* 2011, vol. 30 (1), 95
- **JIN HT ; LAMSA T ; MERENTIE M ; HYVONEN MT ; SAND J ; RATY S ; HERZIG KH ; ALHONEN L ; NORDBACK I.** Polyamine levels in the pancreas and the blood change according to the severity of pancreatitis. *Pancreatology,* 2008, vol. 8 (1), 15-24
- **ZHANG L ; FARRELL JJ ; ZHOU H ; ELASHOFF D ; AKIN D ; PARK NH ; CHIA D ; WONG DT.** Salivary transcriptomic biomarkers for detection of resectable pancreatic cancer. *Gastroenterology,* 2010, vol. 138 (3), 949-57
- **SUGIMOTO M ; WONG DT ; HIRAYAMA A ; SOGA T ; TOMITA M.** Capillary electrophoresis mass spectrometry-based saliva metabolomics identified oral, breast and pancreatic cancer-specific profiles. *Metabolomics,* 2010, vol. 6, 78-95
- **SOGA, T. ; BARAN, R. ; SUEMATSU M. ; UENO, Y. ; IKEDA, S. ; SAKURAKAWA T. ; KAKAZU, Y. ; ISHIKAWA, T. ; ROBERT, M. ; NISHIOKA, T.** Differential methabolomics reveals ophthalmic acid as an oxidative stress biomarker indicating hepatic glutathione sonsumption. *Journal of Biological Chemistry,* 2006, vol. 281 (24), 16768-16776
- **SUGIMOTO et al.** Capillary electrophoresis mass spectrometry-based saliva metabolomics identified oral, breast and pancreatic cancer-specific profiles. *Metabolomics,* 2010, vol. 6, 78-95
- **TSUTSUI et al.** High-throughput LC-MS/MS based simultaneous determination of polyamines including N-acetylated forms in human saliva and the diagnostic approach to breast cancer patients. *Anal Chem,* 2013, vol. 85, 11835-42
- **WANG Q et al.** Investigation and identification of potential biomarkers in human saliva for the early diagnosis of oral squamous cell carcinoma. *Clin Chim Acta.,* 2014, vol. 427, 79-85
- **M. A. HALL.** *Correlation-based Feature Subset Selection for Machine Learning,* 1998
- **MARKO ROBNIK-SIKONJA.** *Igor Kononenko,* 1997
- *Fourteenth International Conference on Machine Learning,* 296-304
- **I. GUYON ; J. WESTON ; S. BARNHILL ; V. VAPNIK.** Gene selection for cancer classification using support vector machines. *Machine Learning,* 2002, vol. 46 (1-3), 389-422
- **BERGER ; JAMES O.** Statistical Decision Theory and Bayesian Analysis. Springer Series in Statistics, 1985
- **D. E. RUMELHART ; G. E. HINTON ; R. J. WILLIAMS.** *Learning representaions by back-propagating errors, Nature,* 1986, vol. 323-9, 533-536
- *J. Platt,* 1998
- Fast Training of Support Vector Machines using Sequential Minimal Optimization. Advances in Kernel Methods-Support Vector Learning
- **YOAV FREUND ; LLEW MASON.** The Alternating Decision Tree Algorithm. *Proceedings of the 16th International Conference on Machine Learning,* 1999, 124-133
- **FREUND, Y. ; MASON, L.** The alternating decision tree learning algorithm. *Proceeding of the Sixteenth International Conference on Machine Learning, Bled, Slovenia,* 1999, 124-133
- **ROSS QUINLAN.** C4.5: Programs for Machine Learning. Morgan Kaufmann Publishers, 1993
- **EIBE FRANK ; IAN H. WITTEN.** Generating Accurate Rule Sets Without Global Optimization. *Fifteenth International Conference on Machine Learning,* 1998, 144-151

- **LINDGREN, F ; GELADI, P ; WOLD, S.** Partial least squares-discriminant analysis; PLS-DA. *The kernel algorithm for PLS. J. Chemometrics,* 1993, vol. 7, 45-59
- **TRYGG, J. ; WOLD, S.** Orthogonal projections to latent structures (O-PLS). *Journal of Chemometrics,* 2002, vol. 16 (3), 119-128
- **BREIMAN, LEO.** Random Forests. *Machine Learning,* 2001, vol. 45 (1), 5-32
- **BREIMAN, LEO.** Bagging predictors. *Machine, Learning,* 1996, vol. 24 (2), 123-140
- **HOTELLING, H.** Analysis of a complex of statistical variables into principal components. *Journal of Educational Psychology,* 1933, vol. 24, 417-441

- **FU NN ; ZHANG HS ; MAM ; WANG H.** Quantification of polyamines in human erythrocytes using a new near-infrared cyanine 1-(epsilon-succinimidyl-hexanoate)-1'-methyl-3,3,3',3'-tetramethyl-indocarbocyanine-5,5'-dis ulfonate potassium with CE-LIF detection. *Electrophoresis,* 2007, vol. 28 (5), 822-9
- **BYUN JA ; LEE SH ; JUNG BH ; CHOI MH ; MOON MH ; CHUNG BC.** Analysis of polyamines as carbamoyl derivatives in urine and serum by liquid chromatography-tandem mass spectrometry. *Biomed Chromatogr,* 2008, vol. 22 (1), 73-80